# DEMANDE DE BREVET EUROPEEN

(11) **EP 1 688 404 A2**
(43) Date de publication de la demande: **09.08.2006**
(21) Numéro de dépôt: 04030916.3
(22) Date de dépôt: 30.04.1997
(51) Int. Cl.: C07B 59/00, C07H 5/02

(54) **Kit comprenant un dispositif de synthèse du 2-[18F]fluoro-2-désoxy-D-glucose (FDG)**

(30) Priorité: 02.05.1996 BE 9600388
(62) Demande divisionnaire de: 97920461.7
(71) Demandeur: GE Medical Systems Benelux SA, 1831 Diegem (Machelen) (BE); UNIVERSITE LIBRE DE BRUXELLES, 1050 Bruxelles (BE); Université de Liège, 4000 Liège (BE)
(72) Inventeur: Damhaut, Philippe, E., 4032 Chenee (BE); Monclus, Michel, 7050 Jurbise (BE); van Namen, John J., 9500 Geraardsbergen (BE); Mulleneers, Eric, 1480 Tubize (BE); Morelle, Jean-Luc E., 1050 Bruxelles (BE); Lemaire, Christian F., 4432 Alleur (BE); Luxen, André, 4560 Ocquier (BE); Lauricella, Benjamin P., 4430 Ans (BE)
(74) Mandataire: Jones Day

(57) **Abrégé**

La présente invention concerne un kit à usage avec un dispositif de synthèse de 2-[¹⁸F]-fluoro-2-desoxy-D-Glucose (FDG), le procédé de synthèse comportant les étapes de: préparation de l'argent de marquage, marguage d'un précurseur sous forme d'un substrat protégé, pré-purification, déprotection et mise en solution finale, caractérisé en ce que l'étage de déprotection, c'est-á-dire la transformation du précurseur marqué en composé marqué, est réalisée directement sur un support solide compris dans une colonne ou dans une cartouche.

## Description

### Objet de l'invention

La présence invention concerne un nouveau procédé de synthèse de composés marqués tels que le 2-[¹⁸F]fluoro-2-désoxy-D-glucose, plus communément appelé fluoro-désoxy glucose ou FDG.

L'invention concerne également un dispositif de synthèse desdits composés marqué qui repose sur ce procédé, rendant possible une opération automatisée incorporant éventuellement un kit d'équipement à usage unique.

### Arrière-plan technologique

Le FDG est un marqueur dont l'usage est en croissance en imagerie médicale nucléaire. Cette molécule marquée avec le radionucléide ¹⁸F se comporte de façon analogue au glucose lors de la première étape de sa métabolisation dans le corps humain et permet de visualiser et de quantifier ce mécanisme fondamental. Elle est indiquée pour le diagnostic de nombreux dysfonctionnements.

La méthode de synthèse du FDG la plus répandue est la méthode dite de Hamacher, qui est décrite dans Hamacher K., Coenen H. et Stöcklin G., Efficient Stereospecific Synthesis of No-carrier-added 2- [¹⁸F]fluoro-2-désoxy-D-glucose Using Aminopolyether Supported Nucleophilic Substitution, Journal of Nuclear Medicine 27, 235 (1986) . Plusieurs variantes de cette méthode ont été mises au point et sont actuellement exploitées dans divers laboratoires de Tomographie par Emission de Positrons (TEP).

La synthèse repose essentiellement sur les étapes opératoires suivantes-:

### Préparation de l'agent de fluoration

Dans une première étape, le ¹⁸F⁻ est activé au moyens d'agents "activateurs" comme le Kryptofix ® (ou K2.2.2) de manière à le rendre plus réactif. Dans certaines publications on les appelle "agents de transfert de phase". Le radionucléide est produit au préalable, généralement par irradiation d'eau enrichie en oxygène-18 avec un faisceau de protons issu d'un accélérateur de particules, sous forme F⁻ (par exemple H¹⁸F en solution aqueuse).

### Marquage du précurseur

Cet agent de fluoration, rendu totalement anhydre par ajouts d'acétonitrile (CH₃CN) et évaporations à sec, est mis en présence d'un substrat de marquage (ou précurseur), généralement le 1,3,4,6-tétra-O-acétyl-2-trifluorométhanesulfonyl-β-D-mannopyranose (plus communément appelé "triflate") solubilisé dans de l'acétonitrile. Une réaction de substitution s'ensuit où le groupement trifluorométhane sulfonate du substrat (c'est-à-dire du "triflate") est remplacé par l'atome ¹⁸F pour former du 2-[¹⁸F]fluoro-1,3,4,6-tétra-O-acétyl-D-glucose (ou tétraacétylfluoroglucose ou TAFg sous la forme abrégée).

### Pré-purification

Les résidus de réactifs, et en particulier, le kryptofix® K2.2.2 sont éliminés en passant la solution à travers un "Sep-Pak® C18" sur lequel le TAFg est fixé. Ce Sep-Pak ® est ensuite rincé avec 10 ml d'HCl 0.1 M. L'activité est ensuite désorbée par 2 ml de tétrahydrofuranne (ou THF) vers le réacteur où celui-ci est totalement éliminé par évaporation.

### Déprotection, en l'occurrence hydrolyse

Le précurseur, en l'occurrence le TAFg, est transformé en FDG par l'élimination de ses quatre groupements acétyles : ces groupements acétyles sont éliminés par une hydrolyse acide réalisée à chaud en solution aqueuse (2 ml HCl 1M à environ 130 °C pendant 15 min.).

### Mise en solution injectable

La solution obtenue, contenant le FDG, est rendue injectable par passage à travers une colonne retardatrice d'ions qui en réduit l'acidité, suivie de Sep-Pak ® alumine et C-18, qui retiennent d'autres impuretés et d'un filtre pour la stérilité. Elle est rendue isotonique par adjonction de NaCl en quantité adéquate. Elle est prête pour le contrôle de qualité ou pour d'autres traitements de préparation à l'administration.

Cette technique comporte cependant une série d'inconvénients, dont les principaux sont :
- la durée de cette procédure est de l'ordre d'une cinquantaine de minutes, notamment en raison du nombre d'opérations de chauffe ou d'évaporation successives, ce qui entraîne une perte d'activité de 30% du simple fait de la demi-vie du ¹⁸F qui est de 110 minutes.
- l'automatisation de cette procédure demande un équipement relativement compliqué et se prête d'autant plus difficilement à la réalisation d'un dispositif à usage unique.

Une étude récente de Mulholland G. Keith (Simple Rapid Hydrolysis of Acetyl Protecting Groups in the FDG Synthesis Using Cation Exchange Resins, Nucl. Med. Biol. Vol. 22, No. 1, pp. 19-23 (1995)) concernant l'hydrolyse a montré que l'acide HCl 1M pouvait être remplacé par une résine cationique présentant des fonctionnalités équivalentes. La résine sulfonique acide sous forme H⁺ (Dowex® 50) préalablement humidifiée, est placée dans le réacteur d'hydrolyse avant de commencer la synthèse. Le tétraacétylfluoroglucose (TAFg) résultant des étapes précédentes de la synthèse, solubilisé dans de l'éther est introduit dans le réacteur, sur la résine. L'éther est évaporé en 3 à 5 minutes. Le réacteur est chauffé à 100 °C pendant 8 à 10 minutes. Au cours de la dernière minute, on ajoute sur la résine 2 ml d'eau. La solution extraite du réacteur contient le FDG directement disponible à un pH de l'ordre de 4 à 5.5.

Cette méthode qui, à notre connaissance, n'est pas exploitée pour l'utilisation *in vivo,* montre que l'hydrolyse acide peut être réalisée à sec sur support solide. Son principal intérêt tient au fait qu'elle rend inutile la purification au travers d'une colonne retardatrice d'ion. En effet, l'acidité de la solution de FDG obtenue est très faible et se situe dans les limites injectables.

La méthode ne représente cependant aucun gain de temps par rapport à l'hydrolyse "classique" de Hamacher (11-15 min). La suppression d'un réactif, l'HCl 1M, pour l'hydrolyse est compensée par l'adjonction de la résine dans le réacteur d'hydrolyse, résine qui de surcroît doit être conditionnée avant usage. Cette méthode maintient la nécessité de chauffage et d'évaporation des solvants.

Une autre étude récente de F. Füchtner et al. (Basic Hydrolysis of 2-[¹⁸F] fluoro-1,3,4,6-tétra-O-acetyl-D-glucose in the Preparation of 2-[¹⁸F]fluoro-2-deoxy-D-glucose, Appl. Radiat. Isot., Vol. 47, No. 1, pp. 61-66 (1996)) a montré que l'hydrolyse, réalisée habituellement en milieu acide, pouvait être réalisée en milieu basique beaucoup plus rapidement et à température ambiante.

La solution résultant de l'étape de marquage est évaporée à sec, ne laissant que le TAFg (et d'autres résidus non volatils) sur les parois du réacteur de marquage. Une solution aqueuse de NaOH (de préférence 2 ml, 0.3M) est transférée dans le réacteur non chauffé. Après 2 minutes, le TAFg est hydrolysé en FDG avec un rendement de l'ordre de 80%. L'avantage principal de ce procédé est la rapidité de la réaction : 2 minutes au lieu de 8 à 10 minutes pour l'hydrolyse acide.

En pratique cependant, le fait que la réaction puisse être réalisée à température ambiante ne constitue pas un avantage dans un dispositif en réacteur, car avant (ou pendant) hydrolyse, il sera de toute façon nécessaire d'inclure un dispositif de chauffage pour évaporer l'acétonitrile (ou l'éther) provenant de l'étape de marquage et qui accompagne nécessairement le TAFg.

### Buts de l'invention

La présente invention vise à fournir un procédé perfectionné permettant de résoudre les difficultés et inconvénients précités, et vise en particulier à :
- réduire la durée et la complexité de la synthèse, et
- simplifier l'appareillage.

Le procédé selon la présente invention a tout particulièrement pour objectif de simplifier la procédure pour rendre plus facile son automatisation et de réduire la durée de la synthèse pour améliorer son rendement, tout en maintenant, voire en améliorant, le rendement chimique du processus de synthèse.

Des avantages spécifiques au procédé et au dispositif de l'invention sont indiqués ci-après en référence à la description de formes d'exécution possibles de l'invention.

### Eléments caractéristiques de l'invention

Le procédé selon l'invention concerne les méthodes de synthèse de composés marqués avec un quelconque élément isotopique et utilisable notamment dans le domaine médical (RMX, thérapie, imagerie médicale, ...), basées sur le marquage d'un substrat organique dont les groupes fonctionnels déterminés sont préalablement protégés au moyen de groupements protecteurs qui, après marquage, peuvent être éliminés facilement par hydrolyse.

Le procédé est caractérisé en ce que l'élimination de ces groupements protecteurs dans l'étape de déprotection est réalisée directement sur un support solide, compris dans une colonne ou cartouche, présentant une forte affinité pour la molécule protégée et une affinité faible pour la molécule déprotégée.

On entend par "groupes fonctionnels", des fonctions telles que alcool, thiol, phénol, thiophénols, amines, cétones, aldéhydes, acides carboxyliques, etc.

On entend par ''groupements protecteurs", des groupements (suivant la fonction à protéger) tels que acétyle, éthers, esters, thicesters, thioéthers, imines, enamines, amides, carbamates, N-Alkyles, N-Aryles, N-hétéro dérivés, cétales, acétales, etc.

On entend par ''colonne" ou "cartouche" toute forme de conditionnement à phase stationnaire utilisable en chromatographie et incluant éventuellement des conteneurs en plastique ou en verre, des colonnes, etc.

Ces produits sont vendus dans le commerce et sont en particulier utilisés en SPE (Solid Phase Extraction) ou chromatographie en phase stationnaire solide.

Selon une forme d'exécution préférée de l'invention, telle qu'appliquée à la synthèse du FDG d'après la méthode de Hamacher, la déprotection est réalisée directement sur la colonne ou cartouche de support solide ayant servi à la pré-purification ou la reformulation du précurseur marqué (élimination des résidus de réactifs et élimination de l'acétonitrile).

La cartouche utilisée peut être par exemple de type C18, C8, tC18, NH2, Diol, polystyrène divinylbenzène (SDB) ou autres phases polymériques telles que disponibles par exemple sous les marques suivantes :
Cartouches Maxi-clean™ de Alltech :
   - cartouche C18 300 mg (Alltech numéro 20922)
   - cartouche C8 300 mg (Alltech numéro 20946)
   - cartouche NH2 300 mg (Alltech numéro 210040)
   Ces cartouches existent également en versions 600 mg et 900 mg.
Cartouches Waters de 50 mg à 10 g, en particulier :
   - cartouches C18 de type Sep-Pak short body (Waters numéro WAT 020 515)
   - cartouches tC18 (trifonctionnelle) de type Sep-Pak short body de 400 mg (Waters numéro WAT 036 810)
   - cartouche d'extraction Waters OASIS HLB
Cartouches Varian :
   - Microbond Elut C18 (référence 1214-4002)
   - Microbond Elut C8 (référence 1214-4405)
   - Microbond Elut PS-SDB (référence 1214-4011)
Cartouches Macherey-Nagel :
   - Chromabond C18 500 mg (référence 730 003)
   - Chromabond Phenyl 500 mg (référence 730 084)

Les cartouches et colonnes utilisées contiennent entre 50 mg et 10 g de support solide. Les quantités préférées sont de 200 à 800 mg. D'autres quantités sont également possibles.

Selon l'invention, le support solide est de phase normale, inverse ou de polarité intermédiaire.

Selon une autre forme d'exécution préférée de l'invention, le support solide est de type phase échangeuse d'ions ou mélange de phases échangeuses d'ions avec une ou plusieurs phases normales ou inverses.

Selon l'invention, le support solide peut être sous forme de grains, de membranes, de feuilles et/ou de capillaires.

Par exemple dans le cas d'une déacétylation comme celle du TAFg, le support solide est de préférence de faible polarité.

En outre, pendant l'étape de déprotection du précurseur, le support solide est avantageusement imprégné par un agent permettant ou accélérant cette déprotection. Cet agent de déprotection peut être une solution aqueuse, de préférence basique, ou une solution aqueuse acide.

Selon une autre variante d'exécution, l'agent de déprotection est le support solide lui-même.

Ladite solution aqueuse basique est de préférence une solution de NaOH et ladite solution aqueuse acide est de préférence une solution de HCl. Selon cette forme d'exécution, le support solide contenu dans la colonne ou la cartouche lors de l'étape de déprotection reste imprégné par la solution acide ou basique pendant 1 à 5 minutes. De plus, on utilise de préférence des concentrations en NaOH ou en HCl dans les solutions basique ou acide comprises entre 0,25 et 2M.

Le précurseur marqué se fixe plus efficacement sur le support solide en milieu aqueux ou en milieu aqueux contenant une proportion faible de solvant organique. Dès lors, la solution de TAFg dans l'acétonitrile (résultant de l'étape de marquage du procédé de Hamacher) est avantageusement diluée avec de l'eau dans une proportion de 10 parties d'eau pour 1 partie de solvant. D'autres dilutions sont également possibles; des dilutions à partir de 5:1 permettent une fixation satisfaisante du TAFg. Il est également apparu qu'il peut être avantageux de diluer la solution de marquage avec une solution acide, par exemple de l'HCl 0,1M, pour améliorer et simplifier l'efficacité de la pré-purification dans le cas où cela est utile, ou toute autre solution aqueuse.

Le mélange est transféré à travers la colonne ou la cartouche de support et ensuite rejeté. Le TAFg, ainsi que certains de ses dérivés, notamment les variétés de TAFg partiellement déacétylés, sont retenus sur le support. Bien entendu, le volume de la colonne ou la cartouche et la quantité de support solide qu'elle contient doivent être suffisants pour retenir l'essentiel du TAFg et de ses dérivés.

La colonne ou la cartouche est ensuite rincée avec une solution aqueuse, soit neutre, soit légèrement acide. Ces rinçages ont pour objectif d'entraîner vers le rejet les traces d'acétonitrile ainsi que de certains résidus de l'étape de marquage présents dans le premier transfert à travers la colonne ou la cartouche.

On notera que le rinçage avec l'HCl 0.1M est facultatif : il dépend de la nature des réactifs utilisés dans l'étape de marquage ou de la composition du diluant ajouté dans la solution marquée avant son transfert à travers la colonne ou la cartouche.

Dans son application à la synthèse du FDG et pour autant qu'un support de type C18 soit utilisé, la procédure décrite jusqu'à ce point est globalement conforme au procédé Hamacher.

Une solution de NaOH est transférée rapidement (quelques secondes) sur la colonne. Son volume est au moins celui que peut contenir la colonne. Le volume transféré sur la colonne peut dépasser le volume de celle-ci, auquel cas l'excès est envoyé au rejet. L'expérience a montré que cet excès n'entraîne qu'une quantité négligeable des composés utiles vers le rejet.

Le support solide reste généralement sous NaOH pendant 1 à 5 minutes. C'est pendant cette période qu'à lieu la déprotection (hydrolyse). La déacétylation complète du TAFg fixé sur le support solide donne lieu au FDG, qui n'a aucune affinité pour le support solide, et se retrouve donc dans la solution qui baigne le support solide. Des concentrations en NaOH comprises entre 0.2M et 2M ont été expérimentées avec succès (concentration optimale variable suivant le support utilisé). D'autres concentrations sont également envisageables, y compris une concentration nulle sur certains supports si l'hydrolyse peut êrre spontanée et rapide, sans ajout de NaOH, ce qui simplifierait considérablement la purification ultérieure.

La molarité en NaOH de la solution et le volume de la colonne définissent la quantité totale de soude dans la colonne ou la cartouche. L'optimisation à ce niveau consiste à réduire cette quantité totale en vue de faciliter plus tard la neutralisation (ou l'élimination) de la soude.

La durée pendant laquelle la colonne ou la cartouche reste remplie de la solution est celle nécessaire pour obtenir une déprotection quasi-complète du TAFg de départ. Elle dépend notamment de la molarité.

Comme alternative à l'utilisation d'une solution de NaOH, une solution d'HCl peut être transférée sur la colonne, et la déprotection peut être également réalisée sur le support solide de manière semblable. La réalisation complète de l'hydrolyse peut demander que la colonne ou la cartouche soit chauffée.

La colonne ou la cartouche est ensuite éluée par un éluant choisi parmi le groupe constitué par de l'eau, une solution aqueuse ou une solution physiologique. Le FDG est entraîné dans la solution. Celle-ci est transférée vers le flacon final au travers d'éléments tels que : colonne retardatrice d'ions, Sep-Pak ® Al₂O₃, C18, filtre destinés à la purifier et à la stériliser.
L'élution peut être effectuée avec une solution de HCl en vue de neutraliser le NaOH (ou l'inverse en cas d'hydrolyse acide). Dans ce cas, l'utilisation d'une colonne retardatrice d'ion est évitée. La mise à pH et isotonicité injectables de la solution finale est assurée par l'ajout d'un tampon. Ce tampon peut être une solution de citrate ou de phosphate de sodium, de tris ou tout autre tampon injectable.

La nature et les dimensions des éléments de purification dépendront notamment de la quantité de soude ou d'acide mis en oeuvre pour réaliser la déprotection (hydrolyse).

Les avantages principaux apportés par la déprotection dans une colonne ou une cartouche de support solide sont les suivants :
- à partir de l'étape de marquage, il n'y a plus aucune évaporation nécessaire;
- aucun solvant intermédiaire n'est utilisé (éthanol dans la synthèse de Hamacher, ou éther dans celle de Mulholland et des fabricants d'équipement CTI ou IBA) pour effectuer la pré-purification;
- la déprotection basique dans la colonne ou la cartouche est rapide et ne demande pas de chauffage;
- on évite l'utilisation d'un réacteur pour la déprotection;
- la quantité totale de base (ou d'acide) nécessaire à la réalisation de la déprotection est réduite au volume de la colonne ou la cartouche. Son élimination est de ce fait simplifiée. Alternativement, sa neutralisation est rendue possible par l'ajout de faibles volumes d'acide (ou de base) et de tampon;
- l'utilisation d'une colonne retardatrice d'ions est évitée.

La déprotection, par exemple par hydrolyse, dans un élément de support solide, et en particulier dans celui utilisé pour la pré-purification, permet donc de raccourcir la durée de la synthèse, de réduire le nombre de réactifs, et de réduire le nombre de composants d'un dispositif de production des composés marqués. Cette simplification rend la technique plus aisément transposable à l'utilisation d'un dispositif de synthèse automatisé incorporant un kit à usage unique.

Un dernier aspect de la présente invention concerne le dispositif de synthèse du 2-[¹⁸F] fluoro-2-désoxy-D-glucose (FDG) par le procédé selon l'invention, dans lequel on utilise un support solide dans l'étape de déprotection, de préférence sous forme d'un kit d'équipement à usage unique.

Avantageusement, ce dispositif est automatisé.

### Description d'une forme d'exécution préférée de l'invention

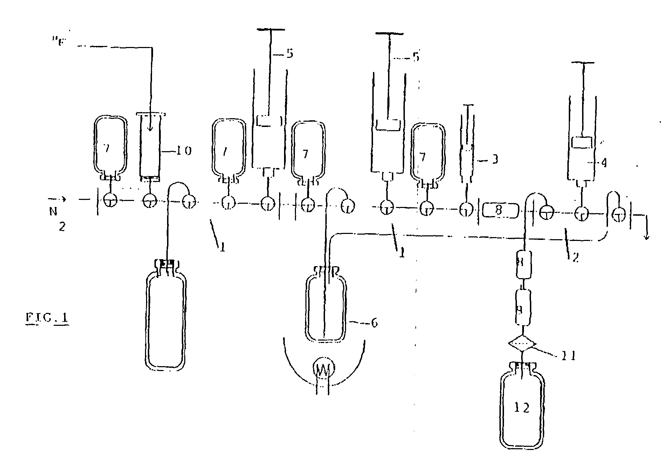

L'invention sera décrite plus en détails en référence à un exemple spécifique d'exécution illustré schématiquement dans la figure 1 annexée.

### Exemple : synthèse de 2-[¹⁸F]fluoro-2-déoxy-D-glucose en utilisant des rampes de vannes et des seringues stériles jetables

La synthèse de 2-[¹⁸F]fluoro-2-désoxy-D-glucose (ou FDG) réalisée dans le module dont le schéma est repris en annexe (figure 1) comprend les étapes suivantes :
- récupération de l'eau enrichie en oxygène 18,
- séchage de l'agent de fluoration [K/222]⁺¹⁸ F⁻,
- marquage du triflate,
- pré-purification,
- déprotection (hydrolyse) en milieu basique sur support solide, et
- formulation de la solution injectable.

Cette synthèse est réalisée dans un kit disposable unique (figure 17 comprenant les composants à usage unique suivants :

| Composants | Marque ou fournisseur | Référence | Qté |
|---|---|---|---|
| Rampe 5 vannes (1) | PVB | 888-105 | 2 |
| Rampe 3 vannes (2) | PVB | 888-103 | 1 |
| Seringue 2 ml (3) | Terumo | BS-02S | 1 |
| Seringue 20 ml (4) | Plastipak | NO. 300134 | 1 |
| Seringue 60 ml (5) | Plastipak | NO. 300856 | 2 |
| Réacteur (6) | Alltech | 66124 | 1 |
| Flacons de réactifs (7) | Alltech | 6655 | 4 |
| Capsule de fixation | Alltech | 66440 | 1 |
| Septum | Alltech | 95313 | 1 |
| Cartouches tC18 (8) | Waters | 36810 | 2 |
| Cartouche alumine | Waters | 20510 | 1 |
| Cartouche SAX (M. PORE) (10) | Varian/3M | | 1 |
| Filtre 0,22 µm (11) | Millipore | SVGS0250S | 1 |
| Fiole stérile sous vide (12) | Mallinckrodt | DRN4370 | 1 |
| Prolongateur | Vigon | 110901 | 2 |
| Aiguille | B. Braun | 466 579/1 | 1 |

## Revendications

1. Un kit à usage avec un dispositif de synthèse FDG automatisé comportant
une rampe comportant
un premier passage pour la réception d'un agent de marquage comprenant du ¹⁸F,
un deuxième passage pour la réception de l'acétonitrile,
un troisième passage pour la réception d'un précurseur comprenant du triflate,
un réacteur en communication liquide avec la rampe, le réacteur étant apte à recevoir, sous contrôle du dispositif automatisé de synthèse FDG ; l'agent de marquage, le précurseur et l'acétonitrile, où un précurseur marqué est produit dans le réacteur ;
une cartouche en communication liquide avec le réacteur, la cartouche comprenant un support solide et étant apte à recevoir, sous contrôle du dispositif automatisé de synthèse FDG ; le précurseur marqué TAFg et un agent de déprotection, où le précurseur marqué est retenu sur le support solide dans la cartouche et une étape de déprotection est exécute directement sur le support solide contenue dans la cartouche, résultant en la production de FDG.

2. Le kit selon revendication 1 **caractérisé en ce que** le kit est un kit à usage unique.

3. Le kit selon revendication 1 ou 2 **caractérise en ce que** chacun des passages comporte une vanne.

4. Le kit selon une des revendications 1 à 3 **caractérise en ce que** la cartouche contient entre 50 mg et 10 g de support solide, de préférence entre 200 et 800 mg.

5. Le kit selon une des revendications 1 à 4 **caractérise en ce que** le support solide dans la cartouche présente une forte affinité pour le précurseur marqué TAFg et une affinité faible pour le FDG.

6. Le kit selon une des revendications 1 à 5 **caractérise en ce que** le kit est apte à utiliser la cartouche sous le contrôle du dispositif de synthèse automatisé de FDG pour (a) l'étape de déprotection et pour (b) une étape de pré-purification dans laquelle la cartouche est rincée avec une solution aqueuse.

7. Le kit selon une des revendications 1 à 6 **caractérise en ce que** le support solide est sous forme de grains, de membranes, de feuilles et/ou de capillaires.

8. Le kit selon une des revendications 1 à 7 **caractérise en ce que** l'agent de déprotection est une solution aqueuse basique ou acide.

9. Le kit selon revendication 8 **caractérise en ce que** la solution aqueuse basique est une solution de NaOH et/ou que la solution aqueuse acide est une solution de HCl.

10. Le kit selon une des revendications 1 à 9 **caractérise en ce que** le kit est apte à fournir à la cartouche, pour l'étape de déprotection, et sous le contrôle du dispositif de synthèse automatisé de FDG, une solution de NaOH d'une concentration comprise entre 0,2 et 2M.
